# EUROPEAN PATENT APPLICATION

(11) **EP 4 729 006 A1**
(43) Date of publication of application: **22.04.2026**
(21) Application number: 25209930.4
(22) Date of filing: 20.10.2025
(51) Int. Cl.: A61B 17/80, A61F 2/28, B33Y 80/00

(54) **IMPLANTS FOR FRACTURE REPAIR**

(30) Priority: 21.10.2024 GR 20240100740
(71) Applicant: Howmedica Osteonics Corp., Mahwah, NJ 07430 (US)
(72) Inventor: CROUS, Heinrich George, Carrigtwohill, T45 RF20 (IE); WALSH, Gearoid, Ennis, V95 X8P2 (IE); CHRISTEN, Alexis, 3360 Herzogenbuchsee (CH); STUCKI, Katja, 5400 Baden (CH); MURPHY, Daniel, Cork, P12 DX51 (IE); DURHAM, James, Cork, P31 XA38 (IE); LOUVIS, Eleftherios, Carrigaline, P43 FX98 (IE); SANCHEZ, Victor, 2607 Cortébert (CH)
(74) Representative: Regimbeau

(57) **Abstract**

An implant system including a bone plate and a flexible implant attachment. The bone plate having a first arm and a second arm extending from a body of the bone plate. The flexible implant having a plurality of links chained together by a plurality of connectors. Each link of the plurality of links includes a core and a set of connectors. The set of connectors of the plurality of connectors extending laterally away from the core. Each connector of the set of connectors defines an opening positioned along a plane that intersects the longitudinal axis of the core at an oblique angle. Each connector of the set of connectors is linked to another connector of the plurality of connectors extending from another link of the plurality of links.

## Description

### BACKGROUND

Bone fractures are often treated through bone reduction in conjunction with fixation via bone plates, bone screws and/or cabling systems. For example, trochanteric fractures are generally fixated with plate systems that may include cable systems. In such cases, due to large variation in the anatomy of the trochanteric region, it is often difficult to tightly fit the implant to the anatomy to sufficiently constrain the fracture and provide sufficient resistance to high loading forces of the abductor muscles attached to the greater trochanter. Because the bone surrounding trochanter fractures is typically very fragile, it can also be difficult to reduce such fractures without causing further damage from point loads and stress concentrations created by fixation components at fragile regions of the bone.

Accordingly, there remains a need for surgical implants capable of providing better stress distribution when deployed to repair bone fractures. There also remains a need for more workable implants that provide a better fit to anatomical features that vary significantly from patient to patient.

### BRIEF SUMMARY

The present disclosure relates to a flexible implant and to an implant system as defined in the appended claims.

In one aspect, the present disclosure relates to a flexible implant that is fabricated as a single assembly having a low profile and capable of connecting to other implants such as new and existing trauma plates. For example, the flexible implant may be configured to attach to a bone plate having a first arm and a second arm extending from a main body of the bone plate. The first arm may include a first connection mechanism, and the second arm may include a second connection mechanism. The first and the second connection mechanisms may be configured to receive and detachably connect with a first end link and a second end link, respectively, of the flexible implant. The first and the second arms may be bendable and extend away from each other in a bifurcated manner as they extend away from the main body of the bone plate. In some instances, at least one of the first and the second arms may include a hook attachment at a respective proximal end of the first or the second arms. The main body of the bone plate may have a tapered profile such that one end of the main body defines an insertion tip.

The flexible implant may include a network of links that form a chainmail construct made of tessellated links that can be interlinked with specialized links. This network of links is a series of interlinked or interconnected links that provide a highly flexible and shape conforming chainmail construct that can mimic any shape the chainmail construct is placed on. Within the chainmail construct there lies a series of specialized links and regions such as interlinked screw holes, cable tunnels, suture loops and spiked links. The chainmail construct is capable of being used as a modular attachment to be fitted with and connected to a trauma plate and work in conjunction with the respective plate to aid in reducing trochanteric fractures and constraining the movement of fractured regions. The chainmail construct of the flexible implant may include a modular clip or modular clips that are adapted to connect the chainmail construct to a bone plate. In such instances, the plate and chainmail construct may be fixed into place through screw fixation, cable supported tightening through cable tunnels or slots and suturing to muscular soft tissue via suture threaded through suture loops. Alternatively, the chainmail construct can also be used as a standalone implant that is also fixed into place through screw fixation, cable supported tightening and suturing to muscular soft tissue. Once implanted, the chainmail construct is fitted over an anatomical region, and the design of the interconnected links allows the chainmail to conform to the anatomical profile that it is resting on to provide equal stress distribution and thereby reduce the risk of point loads and stress concentrations to localized regions of the fragile bone surface.

The chainmail construct of the flexible implant is a self-supporting structure that can be produced using additive manufacturing techniques. The chainmail construct is additively manufactured as a single assembly comprising strong interconnecting pieces or links able to withstand loading forces during implantation and more capable of equally distributing stress across the region of use to reduce stress concentrations that could cause to further damage.

In another aspect, the present disclosure also relates to a trochanter plate designed to have an optimal anatomical fit, the plate having proximal arms that extend from the body of the plate. These proximal arms spread away from each other while extending proximally from the plate to create a U-shape or saddle-like formation. The spread between the proximal arms is such that one of the proximal arms may extend in an anterior direction and the other proximal arm may extend in a posterior direction when the plate is implanted. The trochanter plate includes integrated cable routes extending therethrough, partial cable slots that form peaks and valley structures, screw holes, suture loops and other fixation features use to affix the plate when implanted. The trochanter plate may have a tapered width and a tapered thickness such that the plate has a thin insertion tip at a distal end of the plate, i.e., opposite of the proximal arms. Each of the proximal arms includes a receptacle or cavity configured to receive and attach to a modular clip such as those attached to the chainmail construct mentioned above. For example, the chainmail construct may be designed to fit between or superior to the proximal arms and connect to the arms via the receptacle(s) and the modular clip(s). In such instances, the receptacle or cavity is a female clip that receives and engages with a male clip which is the modular clip. The receptacle or cavity of each of the proximal arms is also configured to receive and attach to an extension arm that includes a malleable hook at its proximal end, and the malleable hook can be bent and twisted through manual manipulation. The extension arm may also include suture loops and partial slots adapted to engage with suture, wire, cable, and the like. The trochanter plate also includes one or more locking mechanism, e.g., a cable lock or crimp, configured to lock and secure the positions of cables, wires and suture. The trochanter plate may be included in a kit with extension arms and/or the flexible implant.

In another aspect, the present disclosure relates to a flexible implant including a plurality of links interlinked by a plurality of connectors. At least one link of the plurality of links may include a link body and a set of connectors of the plurality of connectors. The link body may define a longitudinal axis, and the set of connectors of the plurality of connectors may extend laterally away from the link body. Each connector of the set of connectors may define an opening positioned along a plane that intersects the longitudinal axis of the link body at an oblique angle. Each connector of the set of connectors may be linked to another connector of the plurality of connectors extending from another link of the plurality of links. The set of connectors for at least one link of the plurality of links may include at least two connectors. The at least two connectors of the set of connectors may be parallel to each other. At least one connector of the set of connectors may extend from a first side of a lower portion of the link body to a second side of an upper portion of the link body, and the first side and the second side may be adjacent to each other. The link body of at least one link of the plurality of links may have a height extending along the longitudinal axis ranging from 2 mm to 5 mm. The link body of each of the plurality of links may be a solid cylindrical post. The plurality of links may define a chainmail structure having a tessellated pattern. Each of the plurality of connectors may have the same shape and dimensions. The plurality of links may be fabricated by additive manufacturing as a single-assembly construct such that no assembling of the plurality of links is needed. The plane defined by each connector may be angled such that each connector of the set of connectors is self-supported during and after fabrication.

A first link of the plurality of links may include a screw hole extending through a first link body of the first link along a longitudinal axis of the first link body. The screw hole may include screw engaging members disposed around an inner circumference of the screw hole. At least one link may include a suture loop extending from the link body. A first link of the plurality of links may include a ring extending from a first connector of the first link to a second connector of the first link such that the ring extends above a first link body disposed between the first and the second connectors. A ring may extend between a first link body of a first link of the plurality of links to a second link body of a second link of the plurality of links. The ring may be integral with the first and the second links. The ring may be configured to receive a suture, a wire, or a cable. The opening defined by each connector of the set of connectors may be oblong.

The flexible implant may further include a first end link that has an elongated linkage extending from a link body of the first end link. The first end link may include a set of connectors of the plurality of connectors extending from the first end link away from the elongated linkage. A second end link may be disposed on the opposite side of the flexible implant from the first end link. The elongated linkage may be a modular clip that it is configured to connect to various implants. The plurality links may be movably linked together. The plurality of links are rotatable about each other. The plurality of links may shift laterally with respect to each other.

In some other aspects, the present disclosure relates to an implant system including a bone plate and a flexible implant. The flexible implant may be in the form of a mesh. The bone plate may have a first arm and a second arm extending from a body of the bone plate. The flexible implant may have a plurality of links chained together by a plurality of connectors. At least one link of the plurality of links may include a core defining a longitudinal axis and a set of connectors of the plurality of connectors. The set of connectors may extend laterally away from the core. Each connector of the set of connectors may define an opening positioned along a plane that intersects the longitudinal axis of the core at an oblique angle. Each connector of the set of connectors may also be linked to another connector of the plurality of connectors extending from another link of the plurality of links. The flexible implant may include a first end link and a second end link that are configured to detachable connect to the bone plate. The first arm may include a first connection mechanism. The second arm may include a second connection mechanism. The first and the second connection mechanisms may be configured to receive and detachable connect with the first end link and the second end link, respectively, of the flexible implant. The first and the second arms may extend away from each other as they extend away from the body of the bone plate. At least one of the first and the second arms may include a hook attachment at a respective proximal end of the first or the second arms. The first and the second arms may be bendable such that they can bend to fit around anatomy.

The bone plate may include channels configured to receive surgical cable threaded therethrough. In the bone plate, the bone plate may include suture loop extending therefrom. The bone plate bone may have an uneven surface that is opposite of a contacting surface of the bone plate. The uneven surface may define ridges and grooves that define cable slots. The bone plate may include a plurality of screw holes, each screw hole of the plurality of screw holes disposed between two suture loops. At least one of screw hole of the plurality of screw holes may include screw engaging members extending along the inner circumference of the screw hole. The screw engaging members may be disposed such that they define a diverging and converging profile.

The implant system may further include at least one of suture, bone screws and surgical cable. A link of the plurality of links may have a suture loop extending from the link. A link of the plurality of links may have a screw hole extending through the core of the link. The implant system may further include a pair of arm attachments configured to attach to the bone plate via the first and the second connection mechanisms when the flexible implant is not connected to the bone plate. The first and second connection mechanisms may define a first connect slot and a second connection slot that extend along a first central axis of the first arm and a second central axis of the second arm, respectively. At least one of the first and the second arms may include a first hook arm extension and a second hook arm extension that are configured to be attached to the first and the second arms of the bone plate. The flexible implant may extend between the first and the second hook arm extensions. In other aspects, the present disclosure relates to a bone plate for implanting on a femur bone, the bone plate may include a body portion, a connector portion, and at least one of a first extension arm and a flexible implant. The body portion may extend between a first end and a second end. The connector portion may extend from the second end of the body. The connector portion may be configured for removable engagement with either one of the first extension arm and the flexible implant. The first extension arm may include a first engagement arm configured to be removably engaged with the connector portion. The flexible implant may include a second engagement arm configured to be removably engaged with the connector portion. The connector portion may define a cavity, and the cavity may be configured to receive the first engagement arm or the second engagement arm. The connector portion may include a first connector arm and a second connector arm separate from the first connector arm. The first connector arm may define a first cavity, and the second connector may define a second cavity.

The bone plate may further include a second extension arm that includes a third engagement arm, and the first and the second cavities of the connector portion may be configured to receive the first and the third engagement arms, respectively. The flexible implant may further include a fourth engagement arm disposed on the opposite side of the flexible implant from the second engagement arm, and the first and the second cavities of the connector portion may be configured to receive the second and the fourth engagement arms, respectively. The first cavity may extend along a longitudinal axis of the first connector arm towards the body portion, and the second cavity may extend along a longitudinal axis of the second connector arm towards the body portion. At least one of the first and the second connector arms may include a ridge extending along at least a portion of a length thereof. The first and the second cavities may extend between a first plurality of screw holes defined by the first connector arm and a second plurality of screw holes defined by a second connector arm, respectively, and a central axis of each of the first and the second cavities may be perpendicular to that of the first and the second plurality of screw holes, respectively. The first extension arm and the second extension arm may be included in the bone plate such that the first and the third engagement arms may be disposed within the first and the second cavities of the connector portion, respectively. At least one of the first extension arm and the second extension arm may include a hook. The hook may be malleable such that it is configured to conform to a bone surface. At least one of the first and the second extension arms may include a row of rings extending along a side thereof. A thickness of at least one of the first extension arm and the second extension arm may be tapered. At least one of the first extension arm or the second extension arm may define a reverse curve. The second and the fourth engagement arms may be disposed within the first and the second cavities of the connector portion such that the flexible implant extends from respective terminal ends of the first and the second connector arms.

The flexible implant may further include a fourth engagement arm disposed opposite of the second engagement arm. The second and the fourth engagement arms may be disposed within the first and the second cavities of the connector portion, respectively, and the flexible implant may extend between the first and the second connector arms. The flexible implant may define at least one of a screw hole or a suture loop. The flexible implant may include first region and a second region, and the first region may have a greater degree of flexibility than that of the second region. The second region may be disposed between the first region and an inflexible region defined by either the second engagement arm or the fourth engagement arm. At least one of the first, the second, the third, and the fourth engagement arms may have a V-like shape enclosing a portion of the flexible region. The flexible implant may be additively manufactured. The first and the second connector arms may gradually extend away from each other in a V-like formation. A thickness of the body portion may be tapered along a longitudinal axis of the body portion such that the first end of the body portion defines a thin insertion tip. A width of the bone plate may be tapered along the longitudinal axis of the bone plate such that the first end of the body portion is narrower than the second end.

The bone plate may further include a cable lock that defines at least one central lumen and extends away from the body portion. The cable lock may be configured to hold both ends of a cable that extends through an integrated cable route defined by the bone plate, and the integrated cable route may include at least cable channels and/or partial slots. The connector portion may define a female connector that is adapted to receive a corresponding male connector of either one of the first extension arm and the flexible implant. Either one of the male connector or the female connector may be disposed on one of the first extension arm and the flexible implant. The male connector may define a pair of tabs that may extend from a body portion of the male connector and may be configured to clip into the female connector. The connector portion may define a male connector that is adapted to be received by a corresponding female connector of either one of the first extension arm and the flexible implant. A kit may include one of the various bone plate described herein, a first extension arm, and a flexible implant as described herein.

In yet another aspect, the present disclosure relates to a process for manufacturing a flexible implant. The steps of this process may include additively manufacturing a plurality of support-free links based on a component file. The support-free links may be fabricated without the use of support material. The support-free links may define the flexible implant without any assembling after the additive manufacturing step has been completed. The component file may include a unit cell repeatedly populated within a CAD volume to create a plurality of unit cells that define a digital model of the flexible implant having a tessellated pattern. The support-free links may be additively manufactured as a single assembly of interlinking and movable links. The support-free links of the component file may define a chainmail design. The component file may include specialty links that interlink with the support-free links and define at least one of a screw hole or a suture loop. The specialty links may be self-supporting similar to the support-free links such that no support material is used during the fabrication of the specialty links. The manufacturing process may further include the step of creating the component file that is executable by an additive manufacturing machine. The manufacturing process may further include the step of transferring the component file to an additive manufacturing machine.

In another aspect, the present disclosure relates to a flexible implant having a plurality of links interlinked with each other. Each link from among at least two of the plurality of links include a link body and a set of connectors. The link body may define a longitudinal axis. The set of connectors may extend laterally away from the link body. Each connector of the set of connectors may define an opening positioned along a plane that intersects the longitudinal axis of the link body at an oblique angle, and a connector of the set of connectors of a first link of the at least two links is interlinked to a connector of the set of connectors of a second link of the at least two links. The flexible implant may include a ring extending between a pair of links of the plurality of links. The ring may be integral with each link of the pair of links. The link body may have an elongate dimension aligned along a longitudinal axis, and the elongated dimension may have a height extending along the longitudinal axis ranging, e.g., from 2 mm to 5 mm. The plurality of links may be fabricated by additive manufacturing as a single-assembly construct such that the interlinking between the first link and the second link occurs during the additive manufacturing process. The first link or the second link may include a screw hole extending through the link body of the first link or the second link along a longitudinal axis of the link body. The screw hole may include screw engaging members disposed around an inner circumference of the screw hole. At least one link of the plurality of links may include a suture loop extending from the link body. At least one link of the plurality of links may include a ring extending from a first connector of the at least one link to a second connector of the at least one link such that the ring extends above at least one link body of the at least one link disposed between the first and the second connectors. The ring may extend between a pair of links of the plurality of links. The ring may be integral with each link of the pair of links. At least one link of the plurality of links includes a modular clip configured to connect to other implants. The links of the plurality of links may be movably linked together such that they are rotatable and translatable with respect to each other.

In some other aspects, the present disclosure also relates to various other implant systems. One implant system, for example, may include a bone plate and a flexible implant, which may be in the form of a mesh, comprising a plurality of links. Each link from among at least two links of the plurality of links include a core and a set of connectors extending radially outward from the core. A connector of the set of connectors of a first link of the at least two links may be linked to a connector of the set of connectors of a second link of the at least two links. The plurality of links may include a first end link is configured to detachably connect to the bone plate and a second end link that is configured to detachably connect to the bone plate. As another example, an implant system includes a bone plate having a bendable arm and an arm attachment. The arm attachment may include a modular clip and a flexible implant having a plurality of links interlinked with each other. Each link from among at least two links of the plurality of links may include a body and a set of connectors. In such instances, the set of connectors may extend radially outward from the body. At least one connector of the set of connectors of a first link of the at least two links is linked to a connector of the set of connectors of a second link of the at least two links. The bendable arm may be made from a material that allows the bendable arm to conform to the contours of a patient's bone such that the bendable arm may be partially wrapped around curved regions of the bone.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a top view of a flexible implant with a network of links forming a chainmail structure in accordance with an aspect of the present disclosure;
FIG. 2 is a perspective view of the flexible implant of FIG. 1;
FIG. 3 is a side view of a link of the network of links of FIG. 1;
FIG. 4 is a perspective view of a link of the network of links of FIG. 1;
FIG. 5 is a perspective view of a link of the network of links of FIG. 1;
FIG. 6 is a perspective view of the link of FIG. 3;
FIG. 7 is a partial view of the chainmail structure of FIG. 1;
FIG. 8 is a perspective view of a link with a screw hole in accordance with an aspect of the present disclosure;
FIG. 9 is a perspective view of a link in accordance with an aspect of the present disclosure;
FIG. 10 is a perspective view of a link in accordance with an aspect of the present disclosure;
FIG. 11 is a perspective view of a link in accordance with an aspect of the present disclosure;
FIG. 12 is a top view of a flexible implant with a network of links forming a chainmail structure in accordance with an aspect of the present disclosure;
FIG. 13 is a side view of the chainmail structure of FIG. 11;
FIG. 14 is a perspective view of a flexible implant with a network of links forming a chainmail structure in accordance with an aspect of the present disclosure;
FIG. 15A is a perspective view of a flexible implant with a network of links forming a chainmail structure that is connected to a bone plate in accordance with an aspect of the present disclosure;
FIG. 15B is a perspective view of a flexible implant with a network of links forming a chainmail structure that is connected to a bone plate in accordance with an aspect of the present disclosure;
FIG. 16 depicts a bone plate in accordance with one embodiment of the present disclosure;
FIG. 17 is a partial close-up view of the bone plate of FIG. 16;
FIG. 18 is a top view of a flexible implant attachment compatible with the bone plate of FIG. 16in accordance with one embodiment of the present disclosure;
FIG. 19 is a perspective view of the bone plate of FIG. 16 with the flexible implant of FIG. 18 attached;
FIGs. 20-21 are perspective views of bone plate attachments compatible with the bone plate of FIG. 16 in accordance with one embodiment of the present disclosure;
FIG. 22A is a perspective view of the bone plate of FIG. 16 with bone plate attachments of FIGS. 20 and 21 attached thereto;
FIG. 22B is a partial close up view of the bone plate and bone plate attachment of FIG. 22A;
FIG. 23 depicts a bone plate system in accordance with yet another embodiment of the present disclosure;
FIG. 24 depicts a bone plate system in accordance with one embodiment of the present disclosure;
FIG. 25 is a front perspective view of a bone plate in accordance with another aspect of the present disclosure;
FIG. 26A depicts a bone plate in accordance with an aspect of the present disclosure;
FIG. 26B is a cross-sectional view of a portion of the bone plate of FIG. 26B taken along line A---A';
FIG. 27 is a side view of the bone plate of FIG. 26A;
FIG. 28A is a side view of a bone plate in accordance with an aspect of the present disclosure;
FIG. 28B is a partial close-up view of the bone plate of FIG. 28A;
FIG. 29 is a flow diagram for a process of assembling and implanting implants in accordance with an aspect of the present disclosure; and
FIG. 30 is a flow diagram for a process of assembling and implanting implants in accordance with an aspect of the present disclosure.

### DETAILED DESCRIPTION

As used herein, the term "distal" means more distant from the heart and the term "proximal" means closer to the heart. The term "inferior" means toward the feet and the term "superior" means towards the head. The term "anterior" means towards the front part of the body or the face and the term "posterior" means towards the back of the body. The term "medial" means toward the midline of the body and the term "lateral" means away from the midline of the body.

The specific examples disclosed herein relate to the structural characteristics of various implants along with methods for deploying the same for the specific goal of mitigating further damage when reducing and fixating bone fractures, e.g., trochanteric fractures. Such implants may be implant systems that include plates, hooks, screws, cables, sutures and the like. Examples described herein refer to various types of implants or systems of implants that interconnect with each other. For example, an implant system that includes a trauma plate and a flexible implant or arm attachments, which interconnect with at least one of modular clips, screws, cables, wires, and/or suture. However, the use or non-use of any one of these components is not limited by the use or non-use of the other components as any of the components may be employed individually or separately in a surgical procedure.

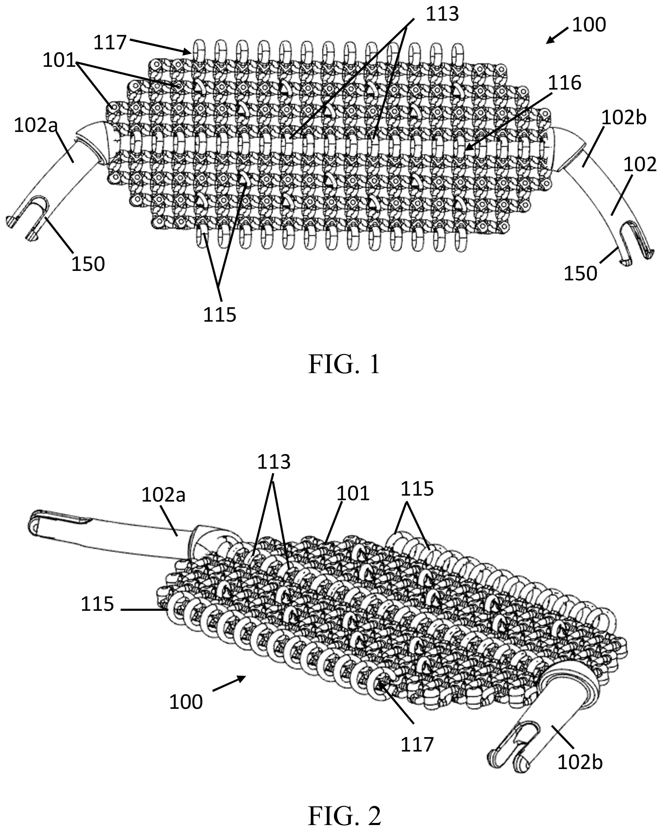

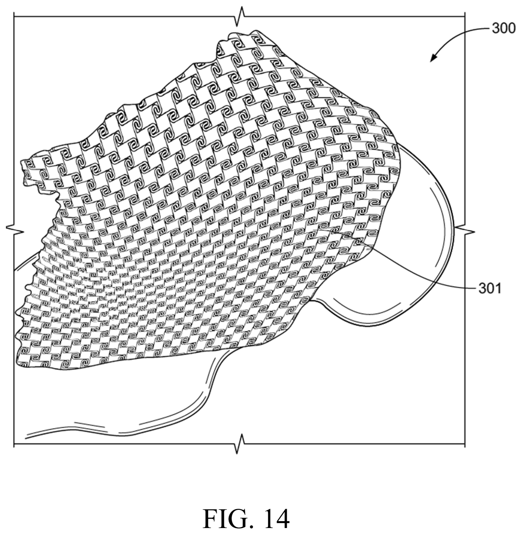

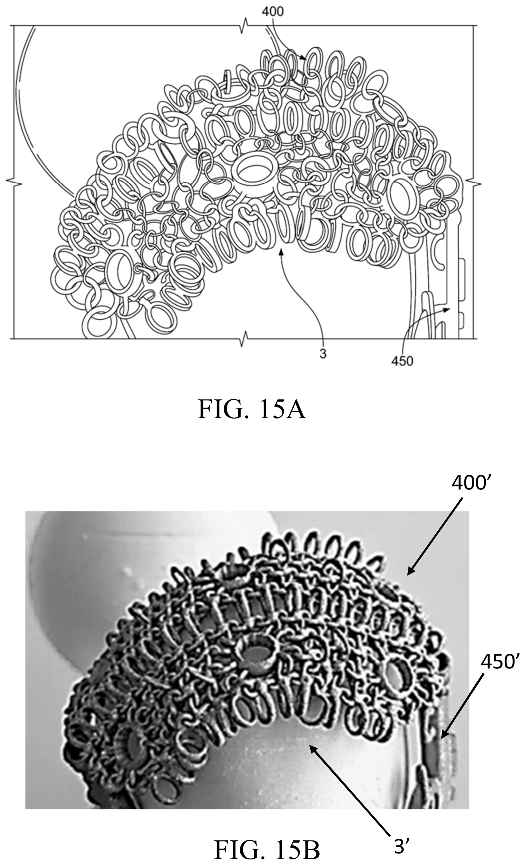

Referring now to the drawings, FIGS. 1-2 show a flexible implant 100 that includes a plexus of links 101 and modular clips 102, i.e., first modular clip 102a and second modular clip 102b, that extend from opposite ends of the flexible implant. Links 101 are interlinked with each other in a way that allows the links to move with respect to each other while in an interlinked state. The flexibility of flexible implant 100, for example, is in part due to links 101 being able to rotate, pivot, shift, translate, and/or twist with respect to each other while remaining interlinked or interconnected with each other. This movement between links 101 allows flexible implant 100 to conform to curved surfaces, e.g., a bone surface, as shown in FIGS. 14 and 15. Links 101 are interlinked together to form a chainmail-like construct having a tessellated pattern, as shown in FIG. 1, that defines a porous structure having openings or voids between the links which may be used to facilitate bone in-growth in certain instances. Links 101 are fabricated in this interlinked state and thereby form a single assembly. This construction not only enhances the mechanical flexibility of the implant, allowing it to adapt to complex anatomical geometries, but also contributes to its structural integrity. The tessellated pattern created by the interlinked links 101 ensures that flexible implant 100 can distribute loads efficiently across its surface, reducing the risk of localized stress concentrations that could lead to material failure or implant deformation. In some embodiments, once interconnected or interlinked, links 101 of flexible implant 100 cannot be unlinked or disconnected without breaking the links.

Modular clips 102a and 102b are adapted to connect with a variety of implant components such as bone plates, screws, rods, and other fixation devices. Each of the modular clips 102a and 102b includes an engagement portion 150, which is adapted to compress and then expand into a cavity of another implant, thereby engaging, i.e., clipping or hooking, to connect with that implant. In this manner, flexible implant 100 is configured to attach to other implants via modular clips 102 as discussed in more detail below.

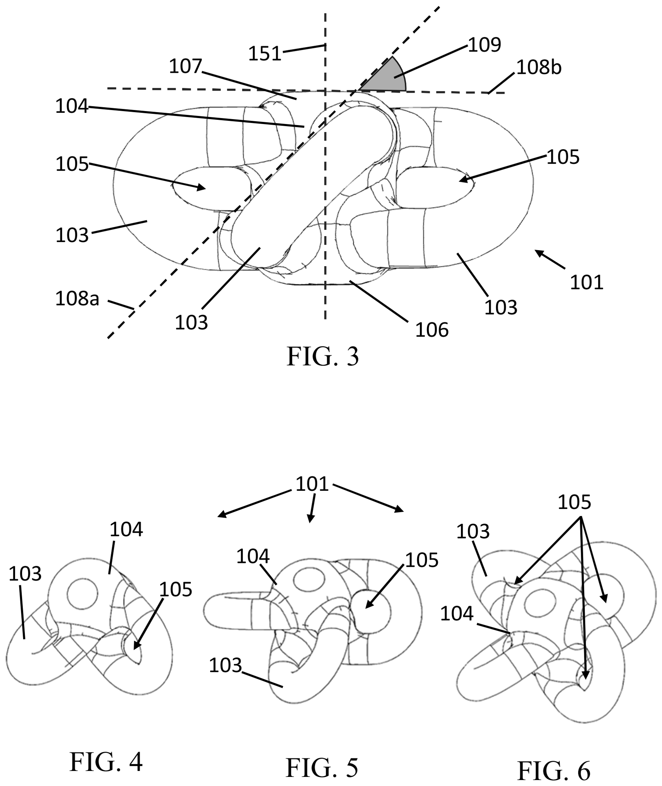

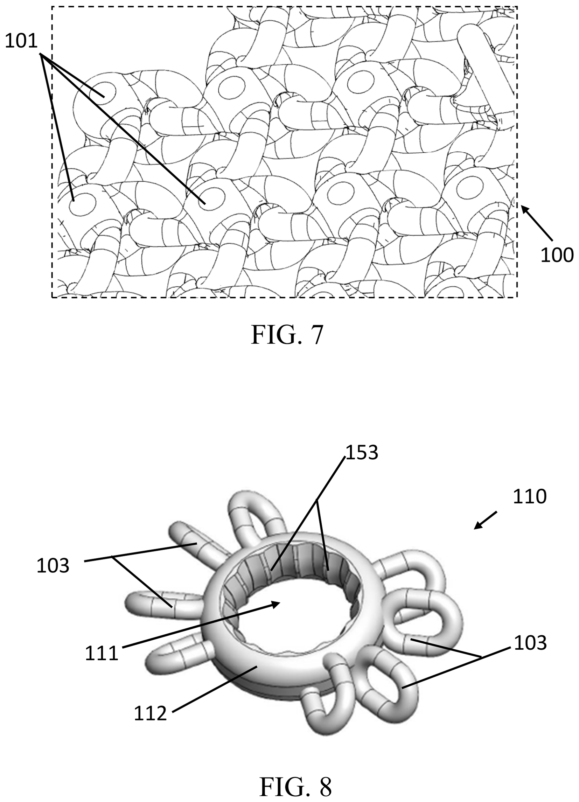

Now referring to FIGS. 3-7, each link 101 has at least one connector 103 extending from it. Some links 101 have two connectors 103, some have three connectors 103, and some have four connectors 103. When there are four connectors 103 on a link 101, the connectors are evenly spaced apart from each other, as shown in FIG. 6. When there are only two or three connectors 103 on a link 101, the connectors are in the same position as when there are four connectors except one or two connectors are missing, as shown in FIGS. 4 and 5. Connectors 103 have a ring-like structure that defines an arc extending from a link body or core 104 such that there is an opening 105 between the link body 104 and each of the connectors. Connectors 103 extend diagonally from a lower region 106 of link body or core 104 of a link 101 to an upper region 107 such that each of the connectors defines an arc that is slanted with respect to link body 104. In other words, each of the connectors is obliquely oriented with respect to the longitudinal axis 151 of the link body, as best shown in FIG. 3. In some instances, each connector 103 defines a diagonal plane 108a that intersects a top plane 108b defined by a top planar surface of link body 104 at an angle 109 that is around 45 degrees, as best shown in FIG. 3. The orientation and configuration of connectors 103 ensure that the links 101 can interlock securely while maintaining the flexibility needed for flexible implant 100 to conform to various anatomical surfaces and connect with other implants. The varying number of connectors 103 on different links 101 allows for customization of the implant's mechanical properties, such as its tensile strength and flexibility, depending on the intended application. The diagonal and oblique orientation of the connectors 103 relative to the link body 104 enables multi-directional movement and load distribution across the implant, reducing stress concentrations that could otherwise lead to material fatigue or failure. Furthermore, the arc-shaped design of the connectors 103, with the defined opening 105, facilitates a robust yet flexible interlinking mechanism, necessary for the implant under dynamic physiological conditions. Each connector 103 on each link 101 is interlinked or interconnected with another connector 103 of another link 101 as shown in FIG. 7. Modular clips 102 also have connectors 103 extending therefrom, which allows the modular clips to attach to the network of links of flexible implant 100. The ability of each connector 103 to interlock with another connector 103 from a different link 101 provides stability to the entire implant system while still allowing for the necessary movement and flexibility. Moreover, the inclusion of connectors 103 on the modular clips 102 extends this interlocking capability, enabling the modular clips 102 to seamlessly integrate with the network of links 101. This integration not only enhances the mechanical continuity of the flexible implant 100 but also ensures that the modular clips 102 are securely anchored within the implant structure, preventing dislocation or detachment during physiological movement.

Links 101 may be fabricated using various processes, some of which are not discussed further herein. In some arrangements, links 101 may be fabricated through an additive manufacturing process, including but not limited to electron beam melting (EBM), selective laser sintering (SLS), selective laser melting (SLM), blown powder fusion for use with metal powders, and the like. Detailed descriptions of the SLS technology may be found in U.S. Pat. Nos. 4,863,538, 5,017,753, 5,076,869, and 4,944,817. Similarly, a detailed description of the use of SLM technology may be found in U.S. Pat. No. 7,537,664. Other methods of additive manufacturing are disclosed in the following references, U.S. Pat. Appl. Publ. Nos. 2006/0147332 A1 ("the '332 Publication") U.S. Pat. Appl. Publ. No. 2011/0014081 A1 ("the '081 Publication"), U.S. Pat. No. 8,992,703 ("the '703 Patent"), U.S. Pat. No. 9,135,374 ("the '374 Patent"), and U.S. Pat. No. 9,180,010 ("the '010 Patent"). The angled orientation of connectors 103 is specifically designed to allow for layer-by-layer fabrication without the need for external support material. In other words, links 101, and specifically connectors 103 of the links, are self-supporting structures that do not need external support during additive manufacturing. As mentioned above, connectors 103 of links 101 are interconnected or interlinked as they are fabricated which means the links cannot be unlinked without breaking one of the connectors. In other words, the fabrication and interlinking links 101 occur simultaneously. In this manner, flexible implant 100 is fabricated as a single-assembly construct and thus requires no assembling of the links to complete the fabrication of the chainmail structure of the flexible implant. Additionally, it is contemplated that modular clips 102 may be fabricated by additive manufacturing techniques, subtractive manufacturing techniques (e.g., CNC milling), or a combination of both. For example, modular clips 102 may be additively manufactured at the same time as links 101. This manufacturing approach offers several advantages. By designing connectors 103 with an angled orientation, the need for additional support structures during the fabrication process is eliminated, which simplifies production and reduces material waste. Furthermore, the ability to fabricate modular clips 102 using the same additive manufacturing process as links 101 allows for the seamless integration of these components into the implant structure. This integrated manufacturing approach ensures that both the links 101 and modular clips 102 are perfectly aligned and fitted, further reducing the risk of mechanical failure or misalignment during surgical implantation. In this manner, the time required to fabricate flexible implant 100 is significantly reduced because removal of support material and assembling components are not needed.

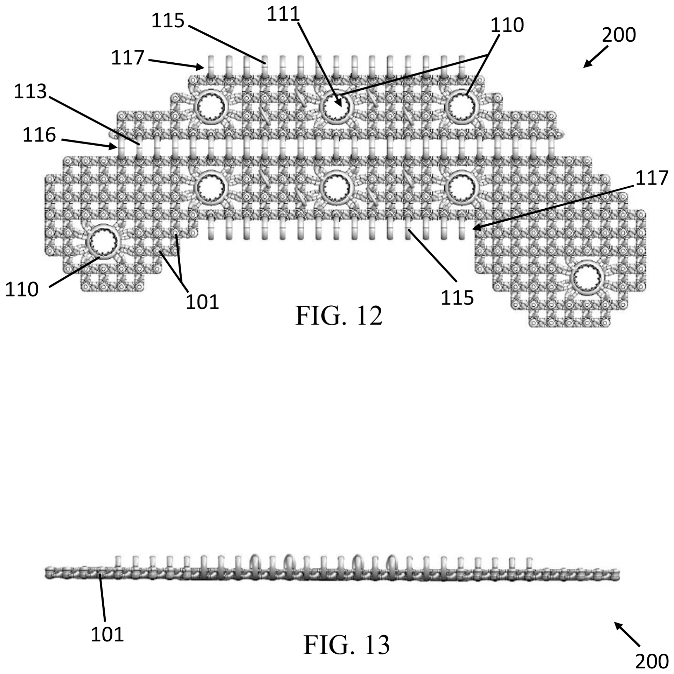

Now referring to FIGS. 8-12, there are a variety of specialty links that interconnect with standard links 101 and feature structural elements designed to engage with various fixation components such as screws, sutures, cables, wires, and the like. For example, as shown in FIG. 8, a screw hole link 110 has an annular structure 112 that defines a screw hole 111, and the screw hole link has screw engaging members 153 extending from an inner surface of the annular structure and connectors 103 extending from an outer surface of the annular structure. Screw hole 111 may range in size between 2.7 mm and 5 mm. Because there are connectors 103 extending from screw hole link 110, this allows for screw hole 111 to be disposed within a flexible implant 200, as shown in FIG. 12, to facilitate fixation of the flexible implant. Specialty links, such as screw hole link 110, improve overall adaptability and functionality of flexible implant 100. Annular structure 112 and screw engaging members 153 are specifically designed to ensure a secure connection with screws, providing stable anchorage points within the implant. The presence of connectors 103 on the outer surface of the screw hole link 110 allows it to be seamlessly integrated into the network of standard links 101, maintaining the flexibility and conformability of the implant while introducing additional fixation capabilities. The variability in screw hole size (ranging from 2.7 mm to 5 mm) offers versatility in accommodating different screw types and sizes, catering to various surgical requirements. This integration within flexible implant 200, as depicted in FIG. 12, not only enhances the mechanical fixation of the implant but also ensures that the implant can be securely attached to underlying bone structures, thereby improving the overall stability and effectiveness of the implant in clinical applications.

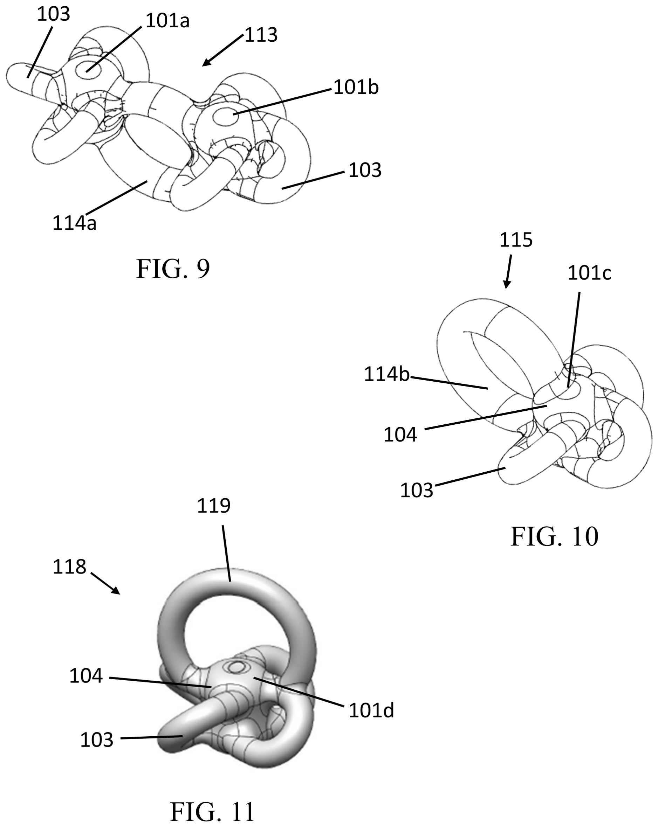

Another specialty link is shown in FIG. 9, which depicts an internal cable link 113 that includes two standard links 101a and 101b that are disposed on opposite sides of cable ring 114a that defines an opening for a cable or wire to be threaded through. The standard links each have three connectors 103 extending therefrom. Because standard links 101a and 101b are located on opposite sides of cable ring 114a, this allows for an internal cable channel 116 to be formed through the chainmail construct of flexible implant 100, 200 as other standard links 101 can link to both sides of the cable ring, as shown in FIGS. 1 and 12.

FIG. 10 shows an external cable link 115 having cable ring 114b and one standard link 101c that are attached together. When multiple external cable links 115 are disposed along an edge of a flexible implant, an external cable channel 117 is formed, which extends along the edge of the implant, e.g., flexible implant 100, 200, as shown in FIGS. 1 and 12. In such instances, link 101c of external cable link 115 interlinks with standard links 101 such that the external cable link becomes a part of the chainmail construct formed by the standard links.

FIG. 11 shows a suture link 118 that has a suture loop 119 extending from a standard link 101d having four connectors 103 extending therefrom. As with the other specialty links, suture link 118 is designed to interlink with standard links 101 via connectors 103 on link 101d and thus become an integral part of the chainmail construct of flexible implant 100, 200. As such, suture loop 119 of suture link 118 provides a passage by which suture may be threaded therethrough and thereby secured to flexible implant 100. In some instances, specialty links can interlink with another specialty. For example, as shown in FIG. 12, screw hole links 110 are interlinked with internal cable links 113 and external cable links 115. These specialty links, namely screw hole link 110, internal cable link 113, external cable link 115, and suture link 118, can be fabricated by additive manufacturing techniques simultaneously with standard links 101. In this manner, the specialty links may be fully integrated and interlinked into a flexible implant, as shown in FIGS. 1 and 12.

The inclusion of these specialty links, such as internal cable link 113 and external cable link 115, enhance the functionality of flexible implant 100, 200 by providing dedicated channels for securing cables and wires, which are necessary for stabilizing and securing the implant during surgery. Suture link 118, with its suture loop 119, offers an additional method for securing soft tissue or other surgical components to the implant, further increasing the versatility of the system. By allowing these specialty links to interlink with both standard links 101 and other specialty links, the implant system ensures that these components are seamlessly integrated, maintaining the structural integrity and flexibility of the overall chainmail construct.

As shown in FIGS. 12 and 13, flexible implant 200 has a low profile, i.e., very slim or narrow side profile, due to the thickness or height of links 101, as best shown in FIG. 13. In some embodiments, links 101 and specialty links described herein may have a thickness or height ranging from about 2 mm to 5 mm and a length and/or a width ranging from about 3 mm to 10 mm. In other embodiments, links 101 and specialty links described herein may have a thickness or height ranging from about 1 mm to 7 mm and a length and/or a width ranging from about 2 mm to 15 mm. Flexible implant 200 includes standard links 101, screw hole link 110, internal cable link 113, and external cable link 115. Flexible implant 200 has a chainmail structure the includes screw holes 111, internal cable channels 116, and external cable channels 117 by having the aforementioned specialty links interlinked with the standard links 101. Internal cable channels 116 comprise multiple internal cable links 113 disposed within the chainmail construct of flexible implant 200 such the respective cable rings 114a of the internal cable links are aligned. External cable channels 117 comprise multiple external cable links 115 disposed within the chainmail construct of flexible implant such the respective cable rings 114b of the external cable links are aligned. In this manner, flexible implant 200 can affixed to bone with screws and cables engaging the screw holes and cables channels defined by the links of the chainmail structure. The low-profile design of flexible implant 200 is particularly advantageous in surgical applications where minimal tissue disruption is essential. The slim profile, determined by the precise thickness and height of the links 101, allows the implant to conform closely to the bone surface without adding unnecessary bulk, which can be critical in areas with limited space or in procedures requiring delicate handling.

Now referring to FIGS. 14, 15A and 15B, flexible implant 300 has a chainmail structure, similar to aforementioned flexible implants 100, 200, that includes links 301 having a squared or cubic shape, as shown in FIG. 14. Additionally, flexible implant 300 is a standalone implant that is not connected to any other implant other than fixation components such as screws, cables, wires, sutures, etc. Alternatively, flexible implant 400, 400' is a component of an implant system where the flexible implant is connected to a bone plate 450,450', as shown in FIGS. 15A and 15B. In this instance, flexible implant 400, 400' is positioned superior to bone plate 450, 450' as the flexible implant is wrapped around the greater trochanteric region 3 of the bone. As a standalone implant, flexible implant 300 is versatile and can be used in various surgical applications where independent fixation is required, providing robust support without the need for additional implants. On the other hand, flexible implant 400, 400', when integrated with bone plate 450, 450', offers a hybrid solution that combines the flexibility and conformability of the chainmail structure with the rigid support provided by the bone plate. This dual-component system is particularly beneficial in complex orthopedic procedures, such as those involving the greater trochanteric region, where both flexibility to conform to anatomical contours and rigid fixation are necessary. The ability of flexible implant 400, 400' to wrap around the bone while being anchored by the bone plate ensures a secure fit, enhancing the overall stability and effectiveness of the surgical repair.

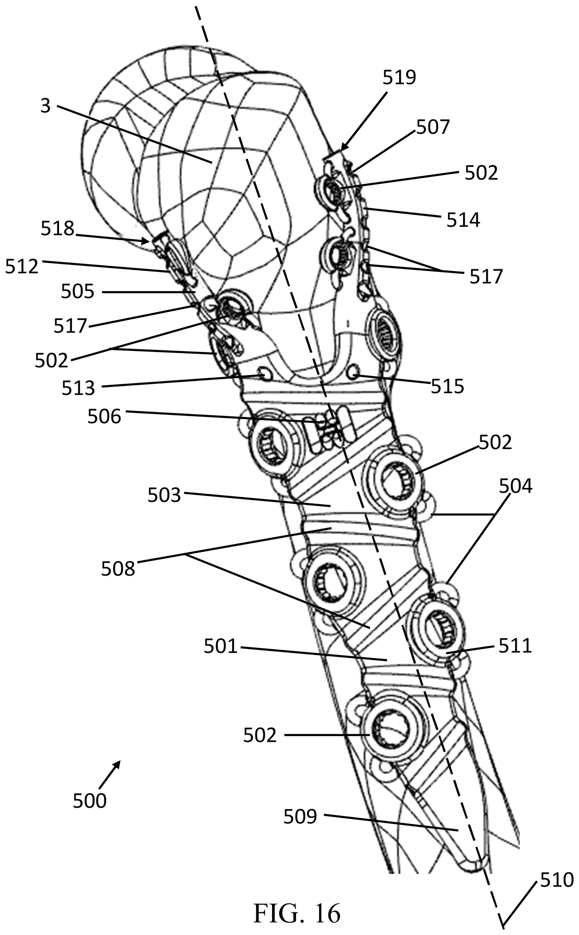

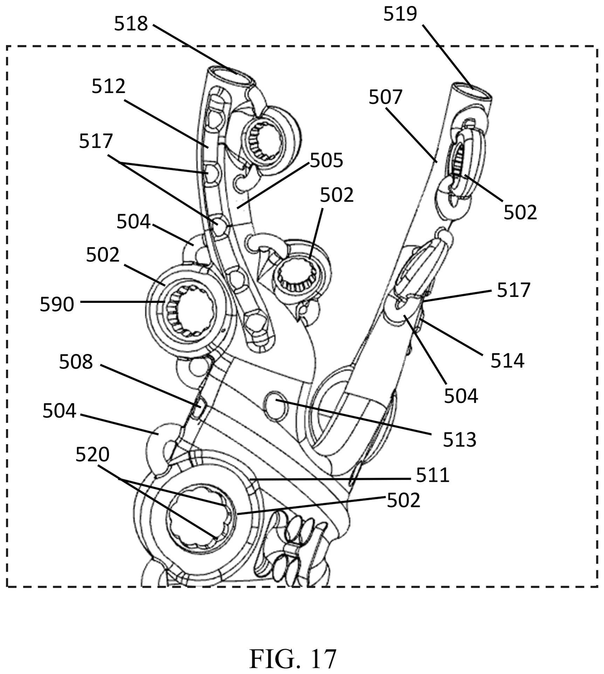

FIGS. 16 and 17 depict an implant in the form of a bone plate 500 according to an embodiment of the present disclosure. Bone plate 500 includes a top surface 501 and a bottom surface or bone contacting surface (not shown) that is opposite to the top surface. In terms of components, bone plate 500 includes a plate body 503, a first appendage or first connector arm 505, a second appendage or second connector arm 507, and a pointed tip 509 opposite the connector arms. A thickness of bone plate 500 is generally defined by the distance between top surface 501 and the bone-contacting surface except in areas where structural features extend beyond these base surfaces.

Plate body 503 includes screw holes 502, suture loops 504, a cable lock 506 and cable channels 508. Suture loops 504 are positioned adjacent to screw holes 502 and between top surface 501 and the bone contacting surface. Cable channels 508 extend across the width of plate body 503 obliquely to the longitudinal axis 510 of bone plate 500. Additionally, at least a portion of cable channels 508 extend beyond top surface 501 and thereby create rib-like features extending across the width of the top surface of plate body 503. Screw holes 502 extend through the thickness of plate body 503 and include an annular structure 511 that extends around each screw hole of screw holes 502 with a raised portion above top surface 501. Annular structure 511 also extends laterally beyond the sides of plate body 503 such that the width of the plate body is extended beyond the bounds of top surface 501 where the annular structures are located. Screw holes 502 further include a counter bore 590 and scalloped sections separated by screw-engaging members 520 spaced apart around the circumference of the screw hole, as shown in FIG. 17. In some examples, the screw holes may have structure as disclosed in U.S. Patent No. 11,039,865. Suture loops 504 extend from a side of an annular structure 511 to a side of plate body 503, as shown, for example, in FIG. 17, to create an opening through which a suture may be inserted. Cable lock 506 extends from plate body 503 and is positioned closer to first and second connector arms 505, 507 than pointed tip 509. Cable lock 506 includes holes through which cable can be threaded and locking or crimping mechanisms arranged to secure or hold the cable therein. The width and thickness of plate body 503 tapers along the length of the plate towards pointed tip 509 such that the pointed tip is the narrowest and thinnest portion of the plate body.

First connector arm 505 and second connector arm 507 also include screw holes 502 each having an annular structure 511, suture loops 504, a first slotted ridge 512 that extends along the length of the first connector arm, and a second slotted ridge 514 that extends along the length of the second connector arm. First and second slotted ridges 512, 514 define slots 517 configured to receive a cable, wire or suture used to secure bone plate 500 to bone or soft tissue. Annular structures 511 that define screw holes 502 extend beyond the sides of first and second connector arms 505, 507, respectively. Suture loops 504, similar to the suture loops connected to plate body 503, extend from a side of an annular structure 511 of a screw hole 502 to a side of either the first or the second connector arms, as best shown in FIG. 17. First connector arm 505 has a first cavity (not shown) that extends internally along the length of the first connector arm such that the first cavity defines a first connector hole 518 at the terminal end of the first connector arm and a first inlet hole 513 at the distal end of the first cavity. The first cavity and first connector hole 518 are configured to receive and attach to a first modular clip or a modular clip (e.g., modular clip 551 discussed below) attached to a flexible implant (discussed in more detail below). Second connector arm 507 has a second cavity (not shown) that extends along the length of the second connector arm such that the second cavity defines a second connector hole 519 at the terminal end of the second connector arm and a second inlet hole 515 at a distal end of the second cavity. The second cavity and second connector hole 519 are configured to receive and attach to a second modular clip or a modular clip (e.g., modular clip 552 discussed below) attached to a flexible implant (discussed in more detail below). The width of first connector arm 505 and second connector arm 507 both taper as the connector arms extend away from plate body 503. First connector arm 505 and second connector arm 507 spread apart from each other as they extend away from plate body 503 and thereby create a U-shaped or saddle-like space between the first and the second connector arms. It is contemplated that first connector arm 505 and second connector arm 507 may be made of a malleable material, e.g., but not limited to, titanium (Ti-6Al-4V) or other titanium alloys, such that they may be able to slightly bend around the anatomy of the patient by manual manipulation. Screw holes 502 that extend through first connector arm 505 and second connector arm 507 have a smaller internal diameter than that of screw holes 502 that extend through plate body 503.

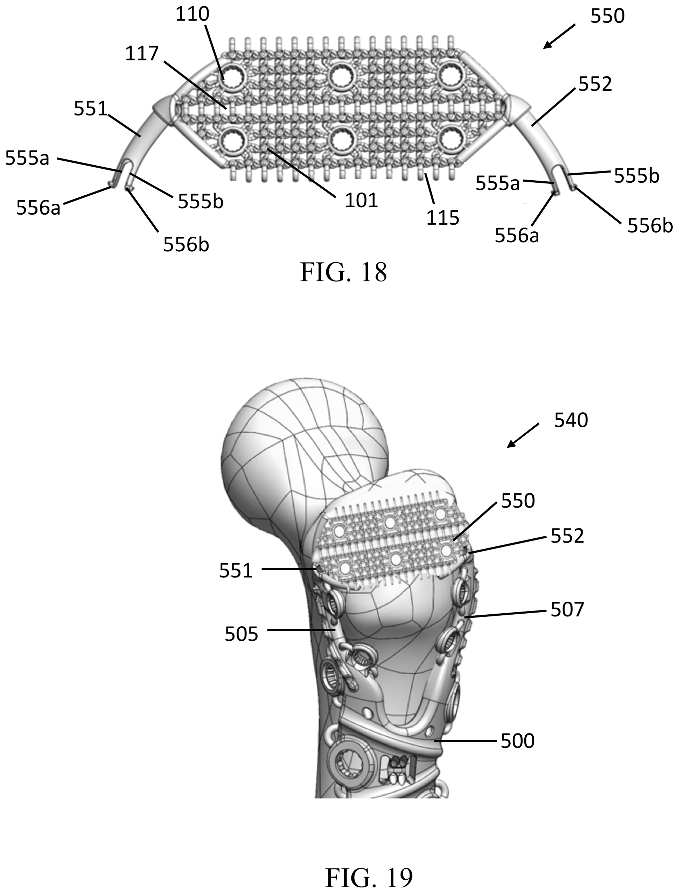

Now referring to FIGS. 18-19, an implant system 540 that includes bone plate 500 and flexible implant 550. When assembled, flexible implant 550 extends between first connector arm 505 and second connector arm 507. Flexible implant 550 is similar to the flexible implants 100, 200, 300, 400 described above in that flexible implant 550 has standard links 101 that are interlinked to form a chainmail structure that includes internal cable channels 116, screw holes 111, and external cable channels 117. Flexible implant 550 also includes a first modular clip 551 and a second modular clip 552 that extend from opposite sides of the flexible implant. First and second modular clips 551, 552 each have two compressible legs 555a, 555b that have a hooked end 556a, 556b designed to engage with a modular clip receptacle (not shown). To attach flexible implant 550 to bone plate 500, first modular clip is disposed within first connector hole 518 of first connector arm 505 that includes a modular clip receptacle (not shown) configured to engage the first modular clip such that the first modular clip is releasably connected to first connector arm 505. Similarly, second modular clip 552 is disposed within second connector hole 519 of second connector arm 507 that includes a modular clip receptacle (not shown) configured to engage the second modular clip such that the second modular clip is releasably connected to second connector arm 507. In this manner, a flexible implant and a bone plate are attached together, which may be done before or after implantation (discussed in greater detail below).

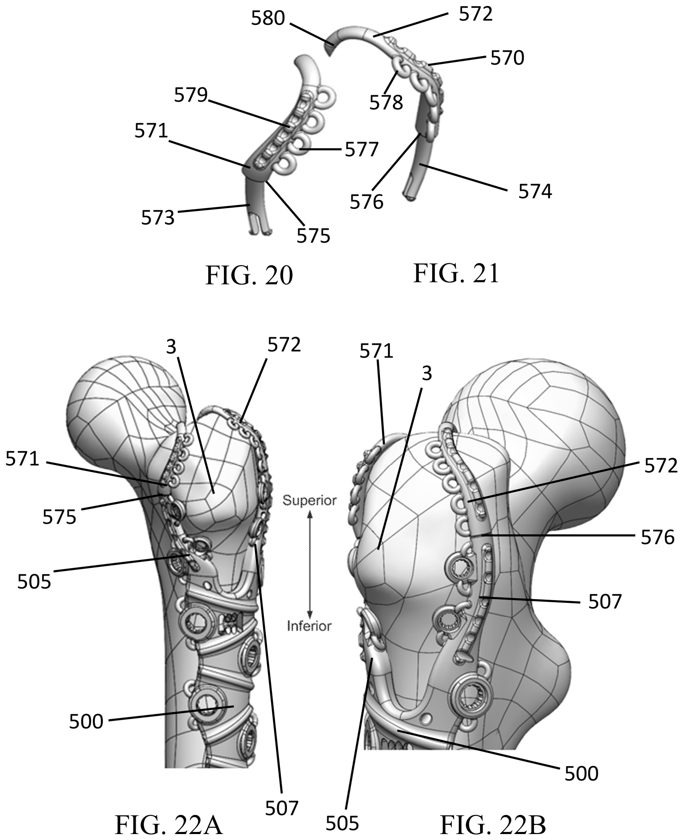

FIGS. 20-22B depict a first extension arm 571 and a second extension arm 572. The first extension arm includes a first modular clip 573 and first lip 575, and the second extension arm includes a second modular clip 574 and second lip 576. First and second modular clips 573, 574 may have a similar structure to modular clips 551, 552, described above. First and second extension arms 571, 572 each define a reverse curve shape, as best shown in FIG. 20, and have a hook or bend 580 at the proximal end of the extension arms, as shown in FIG. 21. First and second extension arms 571, 572 each include a row of suture loops 577, 578 that extend from a respective side of the extension arms. Additionally, a third slotted ridge 579 extends along the length of first extension arm 571, and a fourth slotted ridge 570 extends along the length of the second extension arm 572. It is contemplated that, in some embodiments, first extension arm 571 and second extension arm 572 may include screw holes extending therethrough similar to screw holes 502. First and second extension arms 571, 572 are made from a malleable material, e.g., but not limited to, titanium (Ti-6Al-4V) or other titanium alloys, which can be bent and/or shaped to conform to anatomical features by manual manipulation. In this manner, first extension arm 571 and second extension arm 572 may be altered to wrap around an end of a long bone, e.g., but not limited to, a greater trochanteric region 3 of a femur, such that the extension arms conform to the contours of the bone, as shown in FIGS. 22A and 22B.

FIGS. 22A and 22B depict first extension arm 571 and a second extension arm 572 attached to and extending from first and second connector arms 505, 507, respectively. First modular clip 573 is positioned within, i.e., received by, first cavity (not shown) of first connector arm 505, and second modular clip 574 is positioned within, i.e., received by, second cavity (not shown) of second connector arm 507. The first and second cavities have a modular clip receptacle that is configured to engage first and second modular clips 573, 574. When fully assembled, first lip 575 and second lip 576 are positioned adjacent the respective terminal ends of first connector arm 505 and second connector arm 507.

It is contemplated that alternative embodiments of bone plate 500 may have more or less screw holes 511, suture loops 504, cable channels 508, cable lock(s) 506, and the like than that depicted in the figures. Additionally, in other embodiments, screw holes 511, suture loops 504, cable channels 508 and other features of bone plate 500 may be rearranged and positioned at different locations on the bone plate. For example, in some instances, first and second connector arms 505, 507 may extend from any side of bone plate 500. Furthermore, in one variation of bone plate systems described herein, modular clips 551, 552 (FIG. 18) and modular clips 573, 574 (FIGS. 20-21) may be substituted for other types of clips or connector mechanisms designed to connect flexible implants and extension arms to a bone plate. In still further embodiments, a bone plate 500 may include a single connector arm or three or more connector arms.

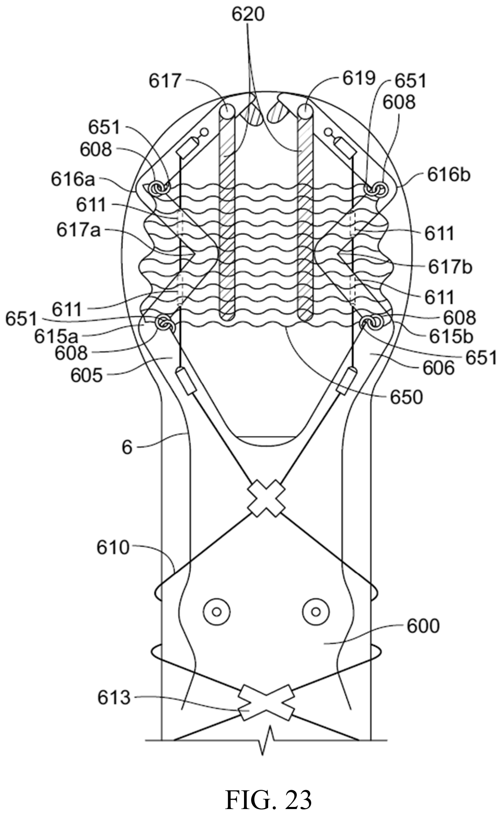

FIG. 23 depicts an implant system 699 fixed to a femur 6 and includes a bone plate 600 having a first arm 605 and a second arm 606, a cable 610, anchor screws 620, and a flexible implant 650. First arm 605 and second arm 606 may each include a connector arm and an extension arm similar to bone plate 500 described above. First arm 605 and second arm 606 bend back and forth in a zig-zag formation and are configured to conform around a proximal end of femur 6. The zig-zag formation of each of first and second arms 605, 606 has a spring-like configuration that is biased in a collapsed state but configured to expand to fit to a patient's anatomy while still maintaining a low profile. In other words, both first and second arms 605, 606 define a first hinge 615a, 615b, a second hinge 617a, 617b, and a third hinge 616a, 616b that are each biased in a folded state (not shown) and configured to expand, as shown in FIG. 23, when pulled to a desired length. In this manner, the spring-like configuration of first and second arms 605, 606 provides a compression force to hold a fractured bone together. Bone plate 600 further includes attachment holes 608 adapted to receive a portion of flexible implant 650. At least one of the attachment holes 608 is positioned at first hinge 615a, 615b and third hinge 616a, 616b in both first arm 605 and second arm 606. In particular, flexible implant 650 includes hooks 651 that are adapted to attach to one of the attachment holes 608. Flexible implant 650, which may be, for example, any one of flexible implant 100, 200, 300 described above, extends between first arm 605 and second arm 606 such that the flexible implant extends across a portion of a femur. Flexible implant 650 may also include screw holes, cable channels, and suture loops such that the flexible implant can facilitate additional fixation of bone plate 600. Flexible implant 650 may be integral with bone plate 600 or separable such that the flexible implant is securely attached to yet detachable from the bone plate. First arm 605 and second arm 606 also include a first screw hole 617 and a second screw hole 619, respectively, that are positioned near the proximal end, i.e., free end of the first and the second arms and arranged to receive one of the anchor screws 620, as shown in FIG. 23, to anchor the first and the second arms to the proximal end of a femur.

With continued reference to bone plate 600, in some arrangements, anchor screws 620 may be long locking screws designed to be screwed into healthy bone in a lesser trochanter or diaphysis region of a femur. Short locking or non-locking screws may be received by respective first arm 605 and second arm 606 to secure the arms to bone. For example, short locking screws may be received in attachment holes 608. Cable 610 extends from the distal end of first arms 605, 606 through cable channels 611 at different locations along the first arm 605 and second arm 606. Cable 610 also extends around the body of bone plate 600 and wraps around a bone. Cable channels or tunnels 611 may be formed inside, on top or through the plate, such that a cable may be threaded therethrough and provide added strength to flexible hook arms. Cable crimps 613 are disposed along the body of the bone plate 600. Cable crimps 613 may be printed inside, on top of or through plate or cable channels or tunnels 611 to allow for bidirectional cable tightening. In this manner, bone plate 600 may be secured to bone by flexible implant, bone screws, cables, and/or sutures. Locking screws 620 may be inserted into first screw hole 617 and second screw hole 619, respectively, which are defined by first arm 605 and second arm 606 such that the screws may access good bone in lesser trochanter/diaphysis.

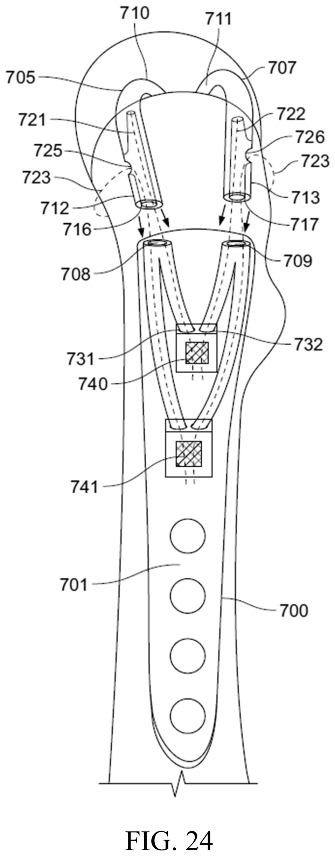

FIG. 24 depicts a lateral view of another implant system, applied to a femoral bone, that includes a first cable 721, a second cable 722, and a third cable 723 (optional) and a bone plate 700 having a plate body 701, a first arm 705 and a second arm 707. Plate body 701 includes a first cavity 708 and a second cavity 709 that are adapted to receive the first arm and the second arm, respectively. First arm 705 and second arm 707 each have a hook-shaped proximal end 710, 711 and an engagement end 712, 713 that is configured to be disposed within first cavity 708 and second cavity 709, respectively. First cable 721 extends from first arm 705 through a first internal channel 716 defined by the first arm to cable lock 740 through first cavity 708 and first hole 731 in bone plate 700, the first hole is disposed between the first cavity and the cable lock. Second cable 722 extends from second arm 707 through a second internal channel 717 defined by the second arm to cable lock 740 through second cavity 709 and second hole 732 in bone plate 700, the second hole disposed between the second cavity and the cable lock. In other words, first cable 721 and second cable 722 extend along a respective path of internal channels defined by bone plate 700 and first arm 705 and second arm 707 until the cables exit first hole 731 and second hole 732, respectively, and then are threaded through cable lock 740. Both first arm cable and 721 and second arm cable 722 may be slidably attached to first arm 705 and second arm 707, respectively. In this manner, the cables securely attach the first and the second arms to the bone plate and allow a distance between the plate body and the first and the second arms to be separately adjusted according to the length of each cable.

Optionally, the third cable 723 may loop around the femur medially such that the third cable may provide stability to the implant system. In such instances, third cable 723 may pass through internal channels and cavities defined by the bone plate 700 to be threaded in a second cable lock 741. For example, first arm 705 and second arm 707 may include a first side opening 725 and a second side opening 726, respectively, which extend into the internal channels of each arm (e.g., first and second internal channels 716 and 717). Third cable 723 may extend through the internal channel of first and second arms 705, 707 and through first and second side openings 725, 726. For instance, third cable 723 may be routed through first internal channel 716, passed through first side opening 725, wrapped around bone, and then routed into second internal channel 717 by being passed through second side opening 726. In this regard, third cable 723 may be used to wrap around bone to secure the bone plate thereto. In this manner, multiple cables may be used in an implant system to secure a bone plate to bone.

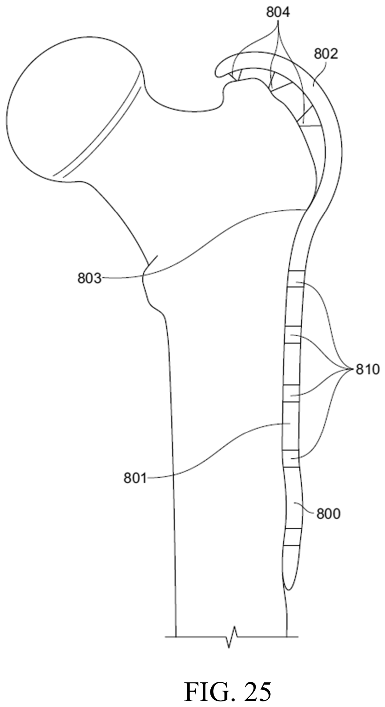

FIG. 25 depicts an anterior-posterior view a bone plate 800, applied to a femoral bone, that includes a plate body 801 and a plate hook 802, the plate body has multiple screw holes 810 disposed along the length of the plate body. Plate hook 802 includes projections 804 extending from a bone contacting side 803 of the bone plate 800. In use, when bone plate 800 is implanted, projections 804 are disposed within corresponding grooves cut into a bone. In this manner, projections 804 act as reference datum points during implantation of the bone plate.

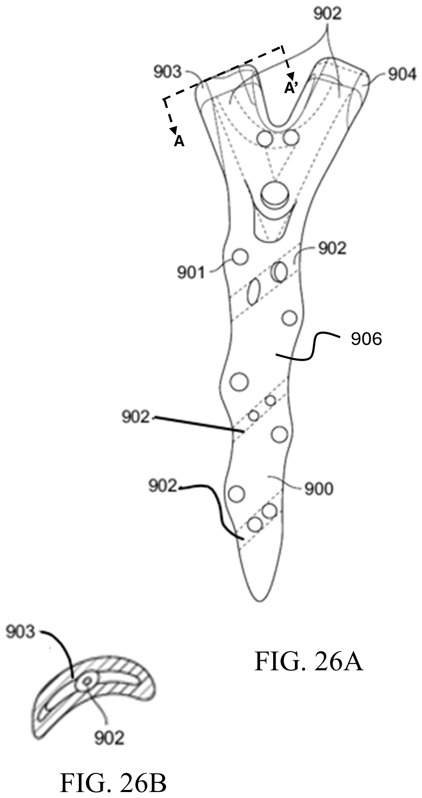

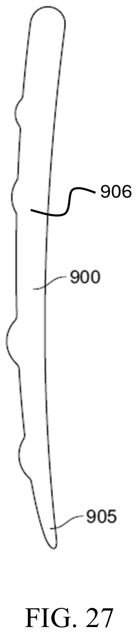

FIGS. 26A-27 depict a bone plate 900 that has a tapering width, as shown in FIG. 26A, and a tapering thickness, as shown in FIG. 27, such that the bone plate has a thin insertion tip 905 at a distal end of the plate. Bone plate 900 also includes a main body 906, a first arm 903, a second arm 904, a plurality of variable angle or poly-axial screw holes 901 extending through the plate, and a plurality of internal cable passages 902 extending through various portions of bone plate 900. First arm 903 and second arm 904 extend from main body 906 in a hook-like manner. Internal cable passages extend diagonally across bone plate 900 between the screw holes. Additionally, internal cable passages extend through first arm 903 and second arm 904, as shown in FIG. 26B.

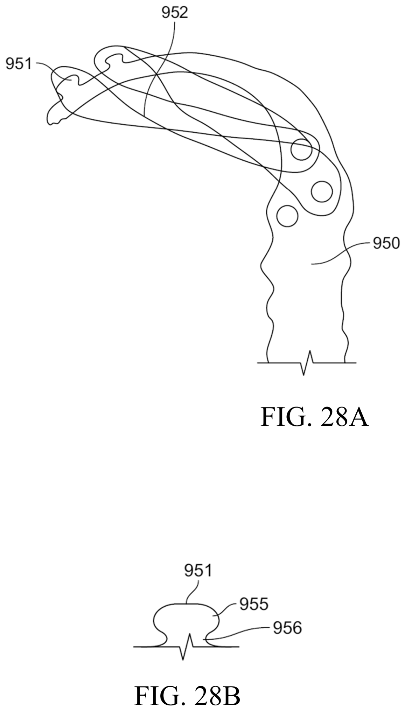

FIG. 28 depicts plate knobs 951 that have a mushroom-like shape with a wide terminal end 955 and narrow neck 956 beneath the terminal end. Knobs 951 may be integral with or detachably attached a plate 950. For example, knobs 951 may be threaded into holes disposed in a plate where needed. In some instances, such holes may be threaded screw holes such that the knobs 951 may be threaded into the screw holes to facilitate cable attachment to plate 950 as opposed to screws. Knobs 951 are arranged such that cable 952 can be weaved around the narrow neck 956 of the knobs and a bone, e.g., the head or neck of a femur, to affix plate 950 to the bone. Cable 952 may be wire or suture that is deployed in a similar manner as wire.

In another aspect, the present disclosure relates to a kit that may include a flexible implant (e.g., flexible implant 100, 200, 300, 400), a pair of extension arms (e.g., first extension arm 705 and second extension arm 707) and a bone plate (e.g., bone plate 500, 600, 700) or plates along with various fixation components such as screws, cables, wires, sutures, and the like. Such kits may be very basic and only include a flexible implant and a bone plate, or a pair of arm attachments and a bone plate. In some embodiments, the kit may include a set of screws, a set of cables, and/or a set of sutures that correspond to the bone plate that is configured to attach a flexible implant and/or a pair of arm attachments. In other embodiments, the kit may only include a flexible implant and fixation components like screws, cables and sutures that are configured to engage the flexible implant to bone. It is contemplated that these kits may further include other surgical tools used in conjunction with the flexible implant(s), bone plate(s), arm attachments and fixation components. In any one of the above embodiments, a kit may include two or more sets of flexible implants and/or pairs of arms. Such additional flexible implants and/or pairs of arms may include flexible implants/pairs of arms that are the same, different or the same and different as others within the kit. In some of the examples stated above, it is contemplated that such kits may include an instruction manual on how to perform one or more of the methods of using the contents of a respective kit.

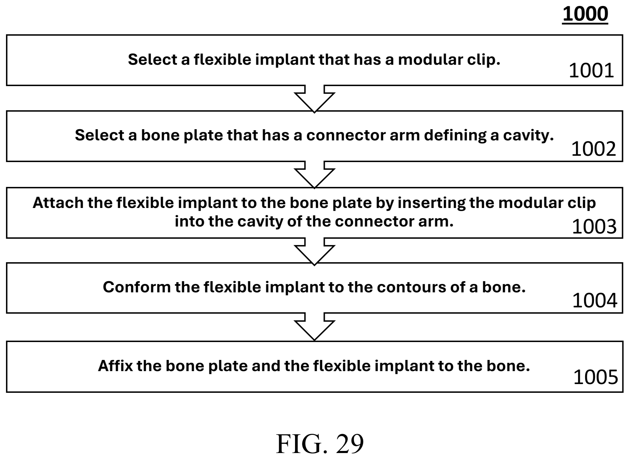

In yet another aspect, the present disclosure relates to a method 1000 of assembling and implanting a flexible implant (e.g., flexible implant 100, 200, 300) with a bone plate (e.g., bone plate 500). To attach the flexible implant to the bone plate, a modular clip (e.g., modular clips 551, 552) of the flexible implant is inserted 1003 into a receptacle or cavity (e.g., first connector hole 518 or second connector hole 519) of a bone plate that is configured to receive and interconnect with the modular clip. In the depicted configurations, a user receives haptic feedback when a connection is made as the clip snaps into place within a respective connector hole. This insertion step 1003 is repeated for any additional modular clips that the flexible implant may have. Next, the flexible implant is conformed 1004 the shape of a bone as the flexible implant is wrapped the contours of the bone. Once the flexible implant is properly positioned, the flexible implant is then affixed 1005 to the bone by inserting screws through screw holes, cables through cable channels, and/or sutures through suture loops that are defined by the chainmail structure of the flexible implant. Additionally, before the flexible implant is attached 1003 to the bone plate, the method may include selecting the flexible implant 1001 and selecting the bone plate 1002, as shown in FIG. 29, in no particular order. In some instances, after the modular clip(s) of the flexible implant are connected to a bone plate, the flexible implant may be oriented with respect to the bone plate, e.g., positioned between to arms of the plate, as shown in FIG. 19. Assembling the arm attachments with the bone plate may be done prior to or after implantation of the plate. It is contemplated that a plate may have been previously implanted in a patient before the arm attachments are later added and attached to the plate in a subsequent surgical operation.

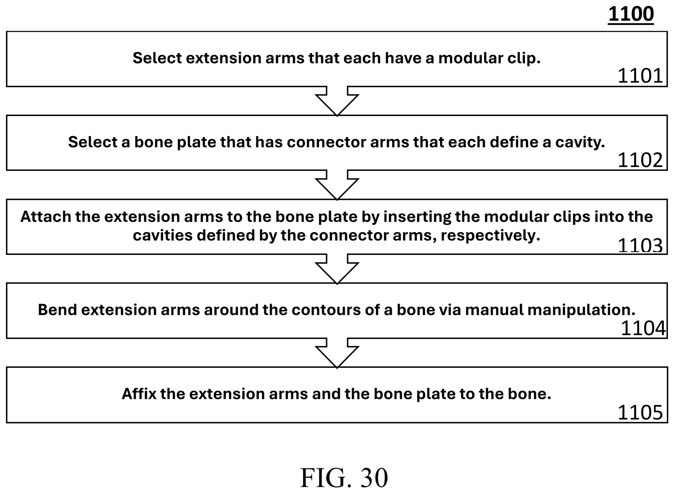

In another aspect, the present disclosure relates to a method 1100 of assembling and implanting a bone plate (e.g., bone plate 500) to a pair of extension arms (e.g., first extension arm 705 and second extension arm 707). To attach the pair of extension arms to the bone plate, a modular clip (e.g., modular clips 573, 574) extending from a first extension arm is inserted 1103 into a receptacle or cavity (e.g., first connector hole 518 or second connector hole 519) of a connector arm of a bone plate that is configured to receive and interconnect with the modular clip. This insertion step 1103 is repeated for a second extension arm. Next, the first and second extension arms are bent 1004 around contours of a bone via manual manipulation. Once the first and the second extension arms are properly positioned, e.g., as shown in FIGS. 22A-22B, the extension arms and the bone plate are then affixed 1105 to the bone by inserting cables through cable channels and slots, sutures through suture loops that are defined by the extension arms and/or screwing screws through screw holes in the bone plate. Additionally, before the extensions are attached to the bone plate, the method may include selecting an extension arm 1101 and selecting the bone plate 1102, as shown in FIG. 30, in no particular order. Assembling the extension arms with a bone plate may be done prior to or after implantation of the plate. It is contemplated that a plate may have been previously implanted in a patient before extension arms are later added and attached to the plate in a subsequent surgical operation.

In yet another aspect, the present disclosure relates to a method of implanting a bone plate. In some specific applications, the bone plate may be used to repair a bone fracture. In one embodiment, a method of bone fixation utilizing a bone plate (e.g., plate 500) begins with abduction of a limb, followed by reduction of bone segments or pieces. Reduction may be completed using a reduction clamp (not shown). Such reduction may be about a joint prosthesis, such as a femoral hip prosthesis, such that the bone segments are reduced adjacent the prosthesis (not shown). For example, the plate is placed along a medial aspect of a femur just inferior to the greater trochanter such that the greater trochanter is at least partially positioned between two connector arms extending from the plate (e.g., first connector arm 505 and second connector arm 507 of plate 500, as illustrated in FIGS. 22a-22b). Once in the desired position, bone screws are placed through screw holes (e.g., screw holes 502) and into the bone. Such bone screws may be polyaxial bone screws so that the screws may be oriented at one of a multitude of angles as desired by the surgeon. Bone screws may be monolithic, biased, unbiased, serrated and/or self-tapping.

Cables, such as Dall-Miles cables, may be pre-threaded through passageways of the plate (e.g., cable channels 508) prior to medial placement of the same, e.g., in a cable lock. Cables may be threaded through passageways in an inferior to superior direction. However, it is also contemplated that the cables may be threaded to the plate in-situ. Cables may be threaded through cable loops or cable channels defined by a flexible attachment (e.g., flexible implant 100, 200, 300, 400, described above). Free ends of the cables are threaded back to a cable lock to tension and hold the cable in place.

In some variations of this embodiment, free ends of each cable may extend from passageways e.g., cable channels 508) and around the bone and back to the plate where they are threaded through openings, cable channels, slots, cable loops, a cable lock (e.g., cable lock 506). Such cables are then tensioned until the trochanteric osteotomy or fracture is reduced and rigidly fixed. The cables are secured, such as by a cable lock, and the remaining free ends of the cables are cut. The plate may include two cables extending horizontally and vertically about the trochanteric bone segment, or one cable extending both horizontally and vertically. As can be appreciated from FIGS. 22A-23, the medial placement of plate inferior to greater trochanter allows cables to be passed over the greater trochanter without interference from a femoral head or neck.

However, in other variations, depending on the nature of the fracture or osteotomy, only horizontal or only vertical cables are passed through passageways. For example, for an extended trochanteric osteotomy, two cables may be passed circumferentially about the femur, without them being routed up and over the greater trochanter. This may be beneficial in cases with poor bone quality. The use of the plate would distribute the cable tension over a greater area than just cables without the plate which might dig in and damage the bone. In some procedures where further securement is desired, bone openings may be drilled through the bone inferior to the bone segment to be rejoined. Cables extending through vertical passageways may then passed over the greater trochanter and down through bone openings about the trochanteric bone segment. In still further embodiments, cables can be routed through both plate at a medial side of a femur and also through a second bone plate placed at another side of femur, such as a standard lateral bone plate. Additionally, strains of suture may be threaded through the passages defined by the plate and then secured around the bone in a similar manner as the cables.

It is to be understood that the disclosure set forth herein includes any possible combinations of the particular features set forth above, whether specifically disclosed herein or not. For example, where a particular feature is disclosed in the context of a particular aspect, embodiment, arrangement, or configuration, that feature can also be used to the extent possible, in combination with and/or in the context of other particular aspects, embodiments, arrangements, and configurations of the technology, and in the technology in general.

Furthermore, although the technology here has been described with reference to particular features and figures, it is to be understood that these features are merely illustrative of the principles and applications of the present technology. It is therefore to be understood that numerous modifications, including changes in the sizes of the various features described herein, may be made to the illustrative arrangement and that other arrangements may be devised without departing from the scope of the claims. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A flexible implant (100, 200, 300, 400, 400', 550, 650) comprising:
a plurality of links (101, 101a, 101b, 101c, 101d, 110, 113, 115, 118) interlinked by a plurality of connectors (103), at least one link comprising:
a link body (104) defining a longitudinal axis (151); and
a set of connectors (103) of the plurality of connectors extending laterally away from the link body (104), each connector (103) of the set of connectors defines an opening (105) positioned along a plane that intersects the longitudinal axis (151) of the link body at an oblique angle, wherein each connector (103) of the set of connectors is linked to another connector (103) of the plurality of connectors extending from another link of the plurality of links.

2. The implant of claim 1, wherein at least two connectors (103) of the set of connectors are in parallel with each other.

3. The implant of any one of claims 1 or 2, wherein the link body (104) of each of the plurality of links (101) is a solid cylindrical post.

4. The implant of any one of claims 1 to 3, wherein the plurality of links (101, 101a, 101b, 101c, 101d, 110, 113, 115, 118) define a chainmail structure having a tessellated pattern such that the links of the plurality of links are movably linked together such that they can rotate and shift laterally with respect to each other.

5. The implant of any one of claims 1 to 4, wherein the plurality of links (101, 101a, 101b, 101c, 101d, 110, 113, 115, 118) is fabricated by additive manufacturing as a single-assembly construct such that no assembling of the plurality of links is needed.

6. The implant of claim 5, wherein the plane is angled such that each connector of the set of connectors is self-supported when the single-single assembly construct is fabricated.

7. The implant of any one of claims 1 to 6, wherein a first link (110) of the plurality of links includes a screw hole (111) extending through a first link body of the first link along a longitudinal axis of the first link body.

8. The implant of claim 7, wherein the screw hole (111) includes screw engaging members (153) disposed around an inner circumference of the screw hole.

9. The implant of any one of claims 1 to 8, wherein the at least one link (118) includes a suture loop (119) extending from the link body.

10. The implant of any one of claims 1 to 9, wherein a ring (114a) extends between a first link body of a first link (101a) of the plurality of links to a second link body of a second link (101b) of the plurality of links, and wherein the ring is integral with the first and the second links.

11. The implant of any one of claims 1 to 10, wherein at least one of links of the plurality of links includes a modular clip (102, 102a, 102b, 551, 552, 573, 574) configured to connect to other implants.

12. An implant system (540, 699) comprising:
a bone plate (500, 600, 700, 900) having a first arm (505, 605, 705, 903) and a second arm (507, 606, 707, 904) extending from a body (503) of the bone plate; and
a flexible implant (550, 650) according to any one of claims 1 to 11, wherein the flexible implant includes a first end link (551, 573) and a second end link (552, 574) that are configured to detachably connect to the bone plate.

13. The implant system of claim 12, wherein the first arm (505, 605) includes a first connection mechanism, and the second arm (507, 606) includes a second connection mechanism, the first and the second connection mechanisms configured to receive and detachable connect with the first end link and the second end link (551, 573, 552, 574), respectively, of the flexible implant (550, 650).

14. The implant system of any one of claims 12 or 13, wherein the first and the second arms (505, 605, 705 ; 507, 606, 707) are bendable and extend away from each other as they extend away from the body of the bone plate.

15. The implant system of any one of claims 12 to 14, wherein at least one of the first and the second arms (705, 707) includes a hook attachment (710, 711) at a respective proximal end of the first or the second arms.
